# EUROPÄISCHE PATENTANMELDUNG

(11) **EP 1 335 203 A2**
(43) Veröffentlichungstag der Anmeldung: **13.08.2003**
(21) Anmeldenummer: 03090035.1
(22) Anmeldetag: 07.02.2003
(51) Int. Cl.: G01N 33/50, C12Q 1/68

(54) **Verfahren und Testbesteck zur Bestimmung von neuroimmunologischen gesundheitlichen Störungen**

(30) Priorität: 09.02.2002 DE 10205587
(71) Anmelder: Bieger, Wilfried P., 86199 Augsburg (DE); Mayer, Wolfgang, 81241 München (DE)
(72) Erfinder: Bieger, Wilfried P., 86199 Augsburg (DE); Mayer, Wolfgang, 81241 München (DE)
(74) Vertreter: Wehlan, Helmut, Dr.

(57) **Zusammenfassung**

Die Erfindung betrifft ein Verfahren und ein Testbesteck zur Bestimmung von neuroimmunologischen gesundheitlichen Störungen wie multiple Chemikalienüberempfindlichkeit (multiple chemical sensitivity - MCS), Sick Building Syndrom (SBS), Chronisches Müdigkeitssyndrom (Chronic Fatigue Syndrom - CFS), Enviromental somatisation syndrome, Fibromyalgie oder Building related illness durch Messung der inflammatorischen oder proinflammatorischen Zytokine wie IFN-gamma und der regulatorischen Zytokine wie IL-10 nach Konfrontation der lymphoiden Zellen mit einem Musterschadstoff oder unter Stimulation mit Recall-Antigen. Bei Patienten mit neuroimmunologischen gesundheitlichen Störungen ist das Verhältnis von inflammatorischen oder proinflammatorischen Zytokinen zu regulatorischen Zytokinen größer als bei Normalpersonen.

## Beschreibung

Die Erfindung betrifft ein Verfahren und ein Testbesteck zur Bestimmung von neuroimmunologischen gesundheitlichen Störungen wie multiple Chemikalienüberempfindlichkeit (multiple chemical sensitivity - MCS), Sick Building Syndrom (SBS), Chronisches Müdigkeitssyndrom (Chronic Fatigue Syndrom - CFS), Enviromental somatisation syndrome, Ideopatic Environmental Intolerance, Fibromyalgie, Building related illness o.ä. sowie von Regulationsstörungen der immunoneuroendokrinen Stressachse, wie z.B. das sog. Burn-Out-Syndrom oder die sog. Sickness-Behaviour (nach Larson et al. 2001, Brain Behaviour Immunity, 15:371-387).

Patienten mit dem Beschwerdebild einer multiplen Chemikalienüberempfindlichkeit (multiple chemical sensitivity MCS) zeigen bei geringer Chemikalienexposition ein obligates systemisches Beschwerdebild. Die betroffenen Personen klagen über Gedächtnisstörungen, Schwäche, multiple Schmerzen im ganzen Körper, Schwindel, Kopfschmerzen, Schweißausbrüche, Atemnot, Zittern, Gelenk- und Muskelschmerzen o.ä.. Diese Reaktion wird durch verschiedenste Substanzen (z.B. Autoabgase, Parfüme, Farben, Lacke, Toner, Insektizide, Holzschutzmittel) ausgelöst, unabhängig von deren chemischer Struktur und bei sehr niedrigen Konzentrationen, die normalerweise auf den Menschen keine subjektiv wahrnehmbaren Auswirkungen haben.

Diese ungewöhnlichen Reaktionen wurden im Laufe der letzten 20 Jahre mit Begriffen wie Sick Building Syndrom (SBS), Chronisches Müdigkeitssyndrom (CFS), Enviromental somatisation syndrome, Fibromyalgie, Building related illness o.ä. umschrieben. Weder sind bis heute die Ursachen dieses Krankheitsbildes bekannt, noch gibt es klare diagnostische Parameter dafür. In der Regel lassen sich bei den betroffenen Personen keine erhöhten Chemikalienspiegel nachweisen. Es sind weder neurologische oder internistische Auffälligkeiten feststellbar, die klassischen Laborparameter sind ebenfalls normal. Ebenso wenig gibt es Anhaltspunkte dafür, dass allergische Reaktionen eine bedeutende Rolle spielen. Immer wieder diskutiert wurde u.a. eine Hyperreaktivität des Immunsystems als Ursache dieses Krankheitsbildes, wobei bisher keine experimentellen oder klinischen Anhaltspunkte für diese Hypothese aufgezeigt werden konnten.

Nach Ashford, N.A. (Toxicology and Industrial Health, 1999, Nr. 3 S. 1-7 und dort zitierte Dokumente) umfasst die Sensitivität gegenüber Chemikalien drei recht unterschiedliche Kategorien:
1) Die Reaktion normaler Versuchspersonen auf bekannte Expositionen entsprechend der herkömmlichen Dosis-Wirkung-Beziehung. Zu dieser Kategorie gehören Reaktionen von Personen am unteren Ende der Verteilung klassischer Reaktionen auf toxische Stoffe innerhalb der Grundgesamtheit sowie die klassische Allergie und andere Formen einer immunologisch vermittelten Sensitivität.
2) Die Reaktion normaler Versuchspersonen auf bekannte oder unbekannte Expositionen, die sich mit klassischen oder bekannten Mechanismen nicht erklären lassen. Zu dieser Kategorie gehören:
   a) das Sick Building Syndrome (SBS), bei dem Menschen auf bekannte oder unbekannte Expositionen reagieren, deren Symptome aber verschwinden, wenn sie der vom Gebäude ausgehenden Belastung nicht ausgesetzt sind; und
   b) die Sensitivität, wie sie beispielsweise durch Toluoldiisocyanat induziert wird, die zunächst als spezifische Überempfindlichkeit für einen einzelnen Stoff (oder eine Stoffgruppe) auftritt, sich aber zu einer unspezifischen Überempfindlichkeit entwickeln kann, wie sie unter der nachstehenden Kategorie 3) beschrieben ist.
3) Die erhöhte übermäßige oder ungewöhnliche Reaktion von Personen auf bekannte oder unbekannte Expositionen, bei denen die Symptome nicht vollständig verschwinden, wenn die Belastung nicht mehr gegeben ist, und/oder bei denen sich die "Sensitivität" auf andere Stoffe auszudehnen scheint. Diese Personen können folgendes zeigen:
   a) eine erhöhte Reaktion auf Stoffe bei gleicher Expositionsdosis im Vergleich zu anderen Personen;
   b) eine Reaktion bei niedrigeren Dosen als diejenigen, die andere Personen beeinträchtigen; und/oder
   c) eine Reaktion zu einem früheren Zeitpunkt als dem für andere Personen zutreffenden Zeitpunkt.

Patienten mit Beschwerden, die in Nordamerika als "Multiple Chemical Sensitivity" (MCS) bezeichnet werden, leiden an der dritten Form von Sensitivität. Ihre gesundheitlichen Probleme sind offenbar häufig (allerdings nicht immer) mit einem zweistufigen Prozess verbunden. Die erste Stufe wird durch eine akute oder traumatische Exposition hervorgerufen, und im Anschluss daran werden bereits bei Belastungen mit Chemikalien in sehr geringer Dosis Symptome ausgelöst und empfindliche Reaktionen beobachtet (zweite Stufe). Die für die Induktion verantwortlichen Chemikalien bzw. Substanzen müssen nicht mit den Stoffen identisch sein, die letztendlich die Reaktionen hervorrufen bzw. auslösen (man spricht gelegentlich auch davon, dass die betreffende Substanz die Person "sensibilisiert", und die Betroffenen werden ebenfalls als "sensibilisiert" bezeichnet.) Es sind nicht in jedem Fall akute oder traumatische Expositionen nötig. Eine Sensibilisierung kann auch durch mehrfache oder durch anhaltende, niedrig dosierte Expositionen hervorgerufen werden.

Oft wird MCS und das Sick Building Syndrom fälschlicherweise als das Gleiche angesehen. MCS differenziert sich jedoch vom Sick Building Syndrome durch das Auftreten von Symptomen in jeder Umgebung, innen oder außen, in der eine betroffene Person gegenüber einer Chemikalie exponiert ist. Gebäudeabhängige Erkrankungen kommen nur in speziellen Innenräumen, wie z.B. Bürogebäuden vor und können zusätzlich auch durch Inhalationsallergene, wie Schimmelpilze unter Umständen gepaart mit Stress am Arbeitsplatz, Temperaturschwankungen, nicht nur durch Chemikalienexposition ausgelöst werden (Kreiss - The epidemiology of building-related complaints and illness, Occup. Med: State of the Art Reviews.1989).

Cullen ("Der Internist", Heft 1/98, S. 105 ff.) definiert MCS wie folgt:
- die Symptome wurden im Zusammenhang mit einer dokumentierbaren Umweltexposition erworben
- die Symptome betreffen mehr als ein Organ
- die Symptome erscheinen und verschwinden in Zusammenhang mit vorhersehbaren Stimuli
- die Symptome werden durch Chemikalien unterschiedlicher Struktur und Wirkmechanismus hervorgerufen
- die Expositionen müssen sehr niedrig sein, im Bereich von 1 % der üblichen Schwellendosis
- kein einzelner Organfunktionstest kann die Symptome erklären.

Das Sick-Building-Syndrom wird auf der Internetseite
(http://www.medizinfo.com/umweltmedizin/syndrome/sickbuilding.htm)
folgendermaßen charakterisiert: Symptome und Verlauf sind Kopfschmerzen, Müdigkeit, Konzentrationsschwäche, Depression, Vergesslichkeit, Empfindungsstörungen an Händen, Füßen, Armen und Beinen, unklare Schmerzen, Reizerscheinungen an Augen, Atemwegen und Haut. Die Symptome klingen im Freien ab und treten in geschlossenen Räumen erneut auf. Ursachen können überheizte Räume, niedrige Luftwechselraten in geschlossenen Räumen, Schimmelpilzsporen, chemische Ausdünstungen, oft auch Klimaanlagen, welche die Luft nur recyceln und nicht erneuern, sein.

Bisher wurde keine somatische Ursache bzw. ein entsprechendes diagnostisches Kriterium für MCS gefunden, so dass vielfach dazu geneigt wird, MCS als psychosomatische Gesundheitsstörung einzustufen.

In den letzten Jahren konnten diagnostisch erstmals Unterschiede zwischen MCS-Patienten, Normalpersonen und Personen mit Depressionen oder anderen psychischen Auffälligkeiten festgestellt werden.

Bisherige diagnostische Feststellungen der MCS oder ähnlicher Erkrankungen umfassen z.B. folgendes:
o Bei Patienten mit Chemikaliensensibilität / -intoleranz wurde demonstriert, dass sie über eine erhöhte nasale Resistenz verfügen gegenüber Normalpersonen, selbst in der Ruhephase (Doty, Deems, Frye, Pelberg, Shapiro - Olfactory sensitivity, nasal resistence and autonomic function in patients with multiple chemical sensitivities. Arch Otolaryngol Head Neck Surg. 1988)
o Neurogene Inflammation in Verbindung mit dem Peptid Substanz P./ Multisystem-Inflammation bei Chemikaliensensitiven (Meggs - Neurogenic inflammation and sensitivity to environmental chemicals. Environ. Health Perspect. 1995)
o Kognitive Abnormalitäten bei der visuellen Erinnerungsfähigkeit bei den Personen mit MCS, die gravierende Veränderungen in ihrer Lebensführung durchführen mussten (Fiedler, Kipen, DeLuca, Kelly-McNeil, Natelson - A controlled comparison of multiple chemical sensitivity and chronic fatigue syndrome, Psychosom. Med. 1996)
o Höhere corticale EEG Aktivierung (geringere Alphawellen und größere Betawellen Aktivität ) bei hochgradig Chemikaliensensiblen (Schwartz, Bell, Dikman, et al. - EEG responses to low level chemicals in normals and cacosmics, Toxicol Ind Health. 1994)
o Perfusionsstörungen durch SPECT-Scans festgestellt, Antikörper gegen Chemikalien (Heuser, Wodani - Multiple chemical Sensitivities: Addendum to biologic Markers in Immunotoxicology National Research Council, National Accademy Press 1992)
o Störungen des Glucosestoffwechsels im Gehirn durch PET festgestellt (Kuklinski-Glutathiontransferasen und Krankheit, ZFU 1999)
o Abnormale Immunzellpopulation, besonders Anstieg von TH1 nach Chemikalienexposition, Autoimmunantikörper (Rea - Chemical Sensitivity Vol. I-IV, 1995)
o Porphyrie, Porphyrinopathie (Donnay, Ziem - Protocol for evaluating disorders of porphyrin metabolism in chemically sensitive patients, MCS Referral & Resources, 1995)
o Abnormale Plasma β-Endorphinwerte (Bell, Bootzin, Davis, Hau, Rithenbaugh, Johnson, Schwartz - Time-Depentent Sensitization of plasma beta-endorphin in community elderly with self-reported environmental chemical odor intolerance, Society of Biological Psychiatry 1996)
o Abnormales Serum Neopterin (Bell, Pataraca, Baldwin, Klimas, Schwartz, Hardin - Serum Neopterin and Somatization in women with chemical intolerance, depressives and normals, Neuropsychobiology 1998).

Das Chronic Fatigue Syndrome ist ein klinisch definiertes Krankheitsbild, das durch schwere, lähmende Erschöpfung und eine Kombination von Symptomen charakterisiert wird, bei denen Konzentrationsschwäche und Merkfähigkeitsstörung, Schlafstörungen, Muskelund Gliederschmerzen im Vordergrund stehen (Holmes GP, Kaplan JE, Gantz NM, Komaroff AL, Schonberger LB, Straus SE et al., Chronic fatigue syndrome: a working case definition, Ann Intern Med. 1988; 108:387-9.).

Spezifische - medizinisch-wissenschaftlich gesicherte - diagnostische Marker zum Nachweis einer CFS gab es bisher nicht.

Da auch bei anderen Krankheiten Symptome - wie ausgeprägte Müdigkeit, Kopfschmerzen u.a. - auftreten können, ist eine sorgfältige Ausschlussdiagnostik bezogen auf bekannte Krankheiten (Differentialdiagnostik) erforderlich (Informationen der Bundesweiten Selbsthilfegruppe für Patienten mit MCS / CFS-Syndrom e.V., Bayreuth/Germany). Durch Laboruntersuchungen müssen deshalb z.B. Entzündungen, Tumore usw. ausgeschlossen werden. Funktionsprüfungen aller inneren Organe, des Pankreas, der Niere, der Leber, des Darms, der Schilddrüse und der Nebenniere sind durchzuführen. Schwere Infektionen an den inneren Organen und im übrigen Körper, wie Tuberkulose, Hepatitis, Colitis, Morbus-Crohn, Oseomyelitis, allergische Erkrankungen können ein ähnliches Müdigkeitssyndrom verursachen.

In der Patentliteratur ist die Diagnose von Krankheiten wie CFS mehrfach beschrieben worden. So wird in der Offenlegungsschrift WO 93/13419 die Anwesenheit des Zytokins Interleukin-1 als Diagnosemarker für CFS verwendet. Auch der lösliche Interleukin-2 Rezeptor, ein Indikator für die Anwesenheit von Interleukin-2, wird als Diagnosemarker eingesetzt (WO 93/14226). In der Schrift WO 97/14963 wird ein Testkit zur Erkennung von CFS bzw. Fibromyalgie auf der Basis eines antipolymeren Antikörpers beschrieben. Eine Diagnose von CFS durch Analyse der peripheren Blutzellen ist Gegenstand des US-Patents 5,538,856. Eine positive Diagnose von CFS liegt dann vor, wenn in CD8+-Zellen die Zell-Marker CD38 oder HLA-DR zunehmen oder die CD11b-Marken abnehmen. Das Dokument WO 96/30759 berichtet über eine Methode zur Diagnose von CFS mittels statistischer Untersuchungen an roten Blutzellen.

Weiterhin wird in der Patentliteratur über Bestimmung eines etwa 30 kDa großen RNase L-Moleküls berichtet (US 5,985,565), das aus peripheren Blutzellen extrahiert wird. Im US-Patent 5,853,996 wird beschrieben, dass in peripheren Blut-Monozyten zur Diagnose von CFS die p68-Kinase-Aktivität, mRNA-Levels, Apoptose und Zell-Zyklus-Analysen bestimmt werden und mit den Werten von gesunden Individuen verglichen werden. Die Diagnose von CFS mittels des 2'-5'A/ RNase L-pathway, einschließlich der Messung des 2'-5'A Oligonucleotid-Levels in peripheren Leukozyten ist Gegenstand der Schrift WO 91/00097.

Auch die Bestimmung eines neuen CFIDS (Chronic Fatigue Immunodysfunction Syndrome) Virus wurde zur Diagnose von CFIDS vorgeschlagen (WO 92/05760). Andere Dokumente berichten über die Bestimmung der spezifischen Dopamin-beta-Hydroxylase-Aktivität (WO 98/58076) oder über Zusammenhänge zwischen Variationen im Arginin-Vasopressin-Receptor-Gen-2 (AVPR2; WO 98/37239).

Die Zytokinsynthese der inflammatorischen und antiinflammatorischen Immunantwort unterliegt einer neuroendokrinen Feed-Back-Regulation. So ist die neuroendokrine Stressreaktion (Cortisol) unmittelbar mit entzündlichen Mechanismen (Anstieg von IFN-gamma, IL1β, IL6, TNF-alpha) assoziiert. Zytokinbestimmungen sind routinegängige Labormethoden, publiziert u.a. von Asadullah et al. 1997, Dtsch.med.Wschr 122:1424-1431.

Zu den wichtigsten Mediatoren der pathologischen Streß-Antwort wird das inflammatorische Zytokin IL1 gerechnet (Konsman et al, Trends Neurosci 25: 154-159, 2002). Es besteht demnach ein neuroendokrin-immunologischer Regelkreis: Nervenzellen weisen Rezeptoren für Zytokine wie IL-1 oder TNF-alpha auf und umgekehrt verrügen Immunzellen über Rezeptoren für biogene Amine wie Adrenalin, Noradrenalin, Dopamin oder Serotonin (Haas, Schauenstein, Allergy 56:470-477, 2001). So stimuliert IL1 die Freisetzung von Cortisol, IFN-gamma hemmt die Serotoninsynthese über die Aktivierung des Enzyms IDO (Indolamin-2,3-Dioxygenase) mit verstärktem Abbau von Tryptophan zu Kynurenin (Munn et al, J Exp Med, 189: 1363-1372, 1999). Unter chronischem Stress nimmt die Ausschüttung von biogenen Aminen ab, auch durch die eingeschränkte Verfügbarkeit von Vorläufermolekülen wie Tyrosin oder Tryptophan. Erkrankungen mit pathologischer Stress-Response wie das Burn-Out-Syndrom oder die sog. Sickness-Behaviour gehen deshalb mit einer inflammatorischen Dysregulation der Immunabwehr einher (Larson et al. 2001, Brain Behaviour Immunity,15:371-387.

Der Nachteil der beschriebenen Lösungen liegt darin, dass es sich um eine Ausschlussdiagnostik handelt, die zwar andere Krankheiten ausschließen kann, aber keinen gesicherten Hinweis auf MCS, CFS, SBS o.a. neuroimmunologische gesundheitliche Störungen liefert. Auch wurde bisher angenommen, dass beispielsweise aufgrund der Unterschiede zwischen MCS und SBS eine einheitliche Diagnose überhaupt nicht möglich ist.

Der Erfindung liegt die Aufgabe zugrunde, die Nachteile der bekannten Lösungen zu beseitigen und neue Möglichkeiten zur Diagnose von neuroimmunologischen gesundheitlichen Störungen, insbesondere schadstoffinduzierten Krankheitsbildern sowie Immunfunktionsstörungen bereitzustellen.

Die Aufgabe wurde dadurch gelöst, dass in lymphozytären oder lymphoiden Zellen von Patienten, die an entsprechenden Krankheiten leiden, die inflammatorischen oder proinflammatorischen Zytokine (Z_{f}) und die regulatorischen Zytokine (Zᵣ) in vitro gemessen werden und deren Verhältnis bestimmt wird. Überraschenderweise hat sich herausgestellt, dass bei betroffenen Patienten in vivo das Verhältnis von (Z_{f}) zu (Zᵣ) größer ist als bei gesunden Kontollpersonen (Normalpersonen). Es wurde ferner überraschenderweise festgestellt, dass Personen mit hochgradiger generalisierter Fremdstoffempfindlichkeit atypisch auf adäquate immunologische Reize reagieren, indem sie in hoher Menge inflammatorische bzw. proinflammatorische Zytokine produzieren. Damit eignet sich das erfindungsgemäße Verfahren erstmals zum Nachweis einer schadstoffinduzierten Immundysregulation der lymphozytären Zellen im Vergleich zu gesunden Kontrollpersonen. Dabei wurden auf Ebene der inflammatorischen (vorzugsweise IFN-gamma) und regulatorischen Zytokine (vorzugsweise IL10) Unterschiede zwischen beiden Gruppen deutlich. Diese proinflammatorische Dysregulation auf Zytokinebene kann durch den Quotienten Proinflammatorische Zytokine (vorzugsweise IFN-gamma, IL2, IL12)/Regulatorische Zytokine (vorzugsweise IL10, IL4, TGF-β) ausgedrückt werden und wird als MCS-Index bezeichnet.

Mit dem erfindungsgemäßen Verfahren können neuroimmunologische gesundheitliche Störungen bzw. Regulationsstörungen der neuroendokrinen Stressachse wie
a) multiple Chemikalienüberempfindlichkeit (multiple chemical sensitivity MCS)
b) Sick Building Syndrome (SBS)
c) Chronisches Müdigkeitssyndrom (CFS bzw. CFIDS)
d) Enviromental somatisation syndrome
e) Ideopatic Environmental Intolerance
f) Fibromyalgie
g) Building related illness
h) Burn-Out-Syndrom
i) Sickness-Behaviour
überraschend klar getestet werden.

Patienten mit neuroimmunologischen gesundheitlichen Störungen weisen demnach in lymphozytären oder lymphoiden Zellen eine erhöhte Induktion an inflammatorischen oder proinflammatorischen Zytokinen oder eine verringerte Kapazität an regulatorischen Zytokinen gegenüber Normalpersonen (Personen die nicht an neuroimmunologischen gesundheitlichen Störungen, insbesondere schadstoffinduzierten Krankheitsbildern sowie Immunfunktionsstörungen leiden) auf. Es konnte festgestellt werden, dass bei erkrankten Patienten in lymphozytären Zellen eine Recall-Antigen- oder Mitogen- induzierte Immunantwort mit proinflammatorischem Grundmuster und verminderter Kapazität an regulatorischen Zytokinen gegenüber Normalpersonen vorhanden ist.

Gemäß einer bevorzugten Ausführungsform der Erfindung werden als inflammatorische oder proinflammatorische Zytokine IFN-gamma, IL-2 und als regulatorische Zytokine IL-10 oder IL-4 gemessen.

Ebenso können die entzündungsrelevanten Monokine der unspezifischen Immunabwehr IL1β (und/oder TNF-alpha, IL6, IL8, IL12) neben den Zytokinen der spezifischen TH1-(IFN-gamma, IL2) und TH2- (IL4, IL5, IL13) sowie regulativen TH3- (IL10, TGF-β) Immunantwort gemessen werden.

Bei Defiziten der spezifischen zellulären Immunkompetenz ist eine verminderte IL2-Induzierbarkeit gegenüber Recall- Antigenen zu beobachten.

Primär ermöglicht die vorliegende Erfindung die Bestimmung von neuroimmunologischen gesundheitlichen Störungen in vitro dadurch, dass lymphozytäre oder lymphoide Zellen von Patienten mit einem Musterschadstoff subtoxischer Konzentration konfrontiert werden und das Verhältnis von (Z_{f}) zu (Zᵣ) bestimmt wird. Dabei werden Musterschadstoffe subtoxischer Konzentration, die bei Normalpersonen keine messbaren Aktivierungsreaktionen auslösen, mit lymphozytären oder lymphoiden Zellen von Patienten mit neuroimmunologischen gesundheitlichen Störungen in vitro inkubiert. Die Verwendung eines generellen Musterschadstoffes zur Feststellung der Chemikalienüberempfindlichkeit ist deshalb möglich, da sich überraschenderweise herausgestellt hat, dass Patienten mit neuroimmunologischen gesundheitlichen Störungen schadstoffunspezifisch eine typische aberrante Immunreaktion im Testsystem zeigen. Diese Reaktion ist charakterisiert durch das Fehlen von IL2 sowie die deutliche Induktion von IFN-gamma und/oder IL10. Dagegen sind alle bisher bekannten immunologischen Unverträglichkeitsreaktionen (Allergien, Autoimmunreaktionen, Pseudoallergien) spezifisch auf einen bestimmten Stoff oder allenfalls noch auf chemisch ähnliche Verbindungen (Kreuzreaktionen) bezogen.

Als Musterschadstoffe kommen z.B. Gemische definierter Zusammensetzungen aus Autoabgasen, Parfümen, Farben, Lacken, Tonern, Insektiziden oder Holzschutzmitteln in Betracht. Die Musterschadstoffe bestehen vorzugsweise aus definierten Zusammensetzungen aromatischer Kohlenwasserstoffen, wie Benzol, Toluol und Xylol.

Ergänzend zum Musterschadstoff können erfindungsgemäß auch Recall-Antigene (Viren, Bakterien, Pilze), Recall-Stimulantien wie LPS (Lipopolysaccharide) oder Mitogene z.B. PWM (pokeweed mitogens) oder PHA (Phytohemagglutinin) eingesetzt werden, für welche die normale physiologische Zytokinexpression in lymphoiden Zellen bei gesunden Personen ermittelt wurde. Dabei können die entzündungsrelevanten Monokine der unspezifischen Immunabwehr IL1β (und/oder TNF-alpha, IL6, IL8, IL12) neben den Zytokinen der spezifischen TH1-(IFN-gamma, IL2) und TH2- (IL4, IL5, IL13) sowie regulativen TH3- (IL10, TGF-β) Immunantwort gemessen werden und damit eine Aussage über deren Kapazität bzw. über Defizite getroffen werden. Zusätzlich hat sich herausgestellt, dass speziell die IL2-Induzierbarkeit gegenüber diesen Recall-Antigenen/Antigenmischungen (z.B. Influenza-Antigene) als ein zentraler Marker der zellulären Immunfunktion im Sinne der Aktivierung von zirkulierenden Gedächtniszellen angesehen werden kann. In Vergleichsuntersuchungen mit dem etablierten in-vitro Verfahren zur Beurteilung der Immunfunktion (Lymphozytentransformationstest, Read-Out Parameter DNS-Neusynthese) zeigte sich eine Übereinstimmung von Zellproliferation nach 5-7 Tagen und der IL2-Induktion nach 6-24 Stunden. Damit kann die antigenspezifische Aktivierbarkeit der zellulären Abwehr über die Messung der initialen IL2-Induktion beurteilt werden.

Damit eignet sich das erfindungsgemäße Verfahren zur Verwendung als Testbesteck zur Bestimmung von neuroimmunologischen gesundheitlichen Störungen sowie von Dysregulationen und Defiziten der TH1/TH2/TH3-Kapazität der zellulären Immunabwehr sowie der Erfassung von Immunfunktionsstörungen.

Das Testbesteck besteht aus mindestens einem Musterschadstoff subtoxischer Konzentration und/oder mindestens einem Recall-Antigen/einer Recall-Antigenmischung, einer Inkubationsvorrichtung für lymphozytäre oder lymphoide Zellen und einer Detektionseinheit für die Erfassung der inflammatorischen oder proinflammatorischen Zytokine und der regulatorischen Zytokine bzw. der Zytokine der unspezifischen, TH1, TH2 und TH3-Immunantwort. Detektionseinheiten sind dem Fachmann prinzipiell bekannt.

Als Recall-Stimulantien Antigene werden Impfstoffe oder Lysate von Viren (z.B. Influenza, EBV, CMV), Bakterien (z.B. Mycobakterien, Streptokokken, Tetanustoxoid ) und Faden-Hefe-oder Schimmel-Pilzen (z.B. Candida, Trichophyton, Mikrosporum, Aspergillus) eingesetzt, insbesondere auch für diese 3 Gruppen (Viren, Bakterien, Pilze) repräsentative Mischungen aus verschiedenen Antigenen. Als unspezifische Stimulantien LPS (Lipopolysaccharide) oder Mitogene werden vorzugsweise PWM (pokeweed mitogens) oder PHA (Phytohemagglutinin) eingesetzt.

Die Messung der Zytokine kann folgendermaßen vorgenommen werden:
a) an isolierten lymphomononukleären Zellen
b) im Vollblut
c) auf mRNA-Ebene mittels quantitativer PCR
d) auf Proteinebene (intrazellulär oder freies Zytokin)
e) indirekt über Transkriptionsfaktoren (NfkappaB, IkappaB)
f) aufgrund genetischer Polymorphismen in den entsprechenden Zytokin-DNA-Sequenzen.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird.

Das Wesen der Erfindung besteht aus einer Kombination aus bekannten (inflammatorische, proinflammatorische und regulatorische Zytokine) und neuen Elementen (das Verhältnis der Zytokine in lymphozytären oder lymphoiden Zellen von Patienten nach Konfrontation mit einem subtoxischen Musterschadstoff bzw. einem physiologischen Stimulanz in Bezug zu neuroimmunologischen Krankheitsbildern oder Immunfunktionsstörungen), die sich gegenseitig beeinflussen und in ihrer neuen Gesamtwirkung einen Gebrauchsvorteil und den erstrebten Erfolg ergeben, der darin liegt, dass erstmals eine positive Diagnose von neuroimmunologischen gesundheitlichen Störungen - im Unterschied zur Ausschlussdiagnostik - bereitgestellt wird. Damit ist ein Nachweisverfahren gefunden worden, mit dem die MCS-Problematik und ähnliche Erkrankungen biochemisch verifiziert werden können.

Die Erfindung soll anhand von Ausführungsbeispielen erläutert werden, ohne auf diese Beispiele beschränkt zu sein.

### Ausführungsbeispiele

### Beispiel 1: Entzündliche in-vivo Situation

Bei MCS-Patienten findet man gegenüber Normalpersonen im peripheren Blut eine deutlich verringerte IL10-Kapazität bei gleichzeitig stark erhöhter IFN-gamma-Induktion. Bei Chemikalienexposition reagieren die MCS-Patienten kaum mit Induktion von IL10, aber mit einer deutlichen Steigerung der IFN-gamma-Induktion.

### Beispiel 2: In-vitro Belastungstest

Es wurden Lymphozyten der Patienten mit einem organischen Stoffgemisch (Benzol-Tolol-Xylol - BTX) inkubiert, das bei Normalpersonen keinerlei messbare Aktivierungsreaktion auslöst. Bei MCS-Patienten fand sich durchweg ein hoher Anstieg von Zytokinen in der Kultur, wobei Gamma-Interferon gegenüber Interleukin 10 dominierte. Bei in-vitro Konfrontation der peripheren lymphoiden Zellen mit einem Musterschadstoff in subtoxischer Konzentration (vorzugsweise BTX) reagieren MCS-Patienten im Vergleich mit Normalpersonen mit einer deutlichen IFN-gamma Induktion (MCS-Index>2).

### Beispiel 3: Proinflammatorische Recall-Antigen-Response

Lymphozytäre Zellen von Patienten mit MCS-Symptomatik zeigen in-vitro im Vergleich zu Normalprobanden (IL2-Dominanz) eine Recall-Antigen-induzierte Immunantwort mit proinflammatorischem Grundmuster (IFN-gamma Dominanz) und verminderter IL10-Kapazität.

## Patentansprüche

1. Verfahren zur Bestimmung von neuroimmunologischen gesundheitlichen Störungen, insbesondere schadstoffinduzierten Krankheitsbildern sowie Immunfünktionsstörungen, **dadurch gekennzeichnet, dass** in lymphozytären oder lymphoiden Zellen von Patienten (in vitro) die inflammatorischen oder proinflammatorischen Zytokine (Z_{f}) und die regulatorischen Zytokine (Zᵣ) bzw. die Zytokine der unspezifischen Immunabwehr oder der TH1, TH2- und TH3- Immunantwort gemessen werden und aus deren Verhältnis die neuroimmunologischen gesundheitlichen Störungen sowie die Immunfunktionsstörungen bestimmt werden.

2. Verfahren nach Anspruch 1, **dadurch gekennzeichnet, dass**
a) in lymphozytären oder lymphoiden Zellen von Patienten mit neuroimmunologischen gesundheitlichen Störungen sowie Immunfunktionsstörungen das Verhältnis von (Z_{f}) zu (Zᵣ) größer als bei Normalpersonen ist.
b) Patienten mit neuroimmunologischen gesundheitlichen Störungen sowie Immunfunktionsstörungen, in lymphozytären Zellen eine Recall-Antigen-induzierte Immunantwort mit proinflammatorischem Grundmuster und verminderter Kapazität an regulatorischen Zytokinen gegenüber Normalpersonen aufweisen.

3. Verfahren nach Anspruch 1 und 2, **dadurch gekennzeichnet, dass** in lymphozytären oder lymphoiden Zellen von Patienten mit neuroimmunologischen gesundheitlichen Störungen sowie Immunfunktionsstörungen eine verminderte IL2-Induzierbarkeit gegenüber Recall-Antigenen bzw. -Stimulantien gemessen wird.

4. Verfahren nach Anspruch 2 und 3, **dadurch gekennzeichnet, dass** als Recall-Antigene Antigenmischungen von Viren, Bakterien oder Pilzen sowie als Recall-Stimulantien LPS (Lipopolysaccharide) oder Mitogene, vorzugsweise PWM (pokeweed mitogens) oder PHA (Phytohemagglutinin) eingesetzt werden.

5. Verfahren nach einem der Ansprüche 1 bis 4, **dadurch gekennzeichnet, dass**
a) als inflammatorische oder proinflammatorische Zytokine IFN-gamma, IL-2, IL1β, TNF-alpha, IL6, IL8 oder IL-12 und als regulatorische Zytokine IL-10 oder TGF-β gemessen werden.
b) als Zytokine der unspezifischen Immunabwehr IL1β, IL6, IL8, IL12, TNF-alpha gemessen werden und als TH1-Zytokine IFN-gamma, IL-2, TNF-alpha, sowie als TH2-Zytokine IL4, IL5, IL13 und als regulatorische Zytokine IL-10 oder TGF-β gemessen werden.

6. Verfahren nach einem der Ansprüche 1 bis 5, **dadurch gekennzeichnet, dass** lymphozytäre oder lymphoide Zellen von Patienten mit neuroimmunologischen gesundheitlichen Störungen sowie Immunfunktionsstörungen und von Normalpersonen in vitro mit mindestens einem Musterschadstoff subtoxischer Konzentration inkubiert werden und das Verhältnis von (Z_{f}) zu (Zᵣ) bestimmt wird bzw. die Zytokine der unspezifischen Immunabwehr oder der TH1, TH2- und TH3- Immunantwort gemessen werden.

7. Verfahren nach Anspruch 6, **dadurch gekennzeichnet, dass** nur die lymphozytären oder lymphoiden Zellen von Patienten mit neuroimmunologischen gesundheitlichen Störungen sowie Immunfunktionsstörungen - nicht aber Normalpersonen - gegenüber dem Musterschadstoff eine deutliche IFN-gamma und/oder IL10-Induktion, aber keine IL2-Induktion aufweisen.

8. Verfahren nach Anspruch 6 und 7, **dadurch gekennzeichnet, dass** als Musterschadstoffe Gemische definierter Zusammensetzungen
a) aus Autoabgasen, Parfümen, Farben, Lacken, Tonern, Kohlenwasserstoffen, Lösemitteln, Insektiziden oder Holzschutzmitteln
b) aus aromatischen Kohlenwasserstoffen.
c) aus Benzol, Toluol und Xylol.
eingesetzt werden

9. Verfahren nach einem der Ansprüche 1 bis 8, **dadurch gekennzeichnet, dass** die Messung der Zytokine folgendermaßen erfolgen kann:
a) an isolierten lymphomononukleären Zellen
b) im Vollblut
c) auf mRNA-Ebene mittels quantitativer PCR
d) auf Proteinebene (intrazellulär oder freies Zytokin)
e) indirekt über Transkriptionsfaktoren (NfkappaB, IkappaB)
f) aufgrund genetischer Polymorphismen in den entsprechenden Zytokin-DNA-Sequenzen.

10. Verfahren nach Anspruch 1 bis 9, **dadurch gekennzeichnet, dass** es sich bei den neuroimmunologischen gesundheitlichen Störungen, insbesondere schadstoffinduzierten Krankheitsbildern sowie Immunfunktionsstörungen, um
a) multiple Chemikalienüberempfindlichkeit (multiple chemical sensitivity MCS)
b) Sick Building Syndrome (SBS)
c) Chronisches Müdigkeitssyndrom (CFS bzw. CFIDS)
d) Enviromental somatisation syndrome
e) Ideopatic Environmental Intolerance
f) Fibromyalgie
g) Building related illness
j) Burn-Out-Syndrom
k) Sickness-Behaviour
handelt.

11. Testbesteck zur Bestimmung von neuroimmunologischen gesundheitlichen Störungen und Immunfunktionsstörungen gemäß Anspruch 1 bis 10, bestehend aus mindestens einem Musterschadstoff/einer Musterschadstoffmischung subtoxischer Konzentration und/oder mindestens einem Recall-Antigen/einer Recall-Antigenmischung, einer Inkubationsvorrichtung für lymphozytäre oder lymphoide Zellen und einer Detektionseinheit für die Erfassung der inflammatorischen oder proinflammatorischen Zytokine und der regulatorischen Zytokine bzw. der Zytokine der unspezifischen, TH1, TH2 und TH3-Immunantwort.

12. Testbesteck nach Anspruch 1, **dadurch gekennzeichnet, dass** es als Recall-Stimulantien Antigene, Impfstoffe oder Lysate von Viren (z.B. Influenza, EBV, CMV), Bakterien (z.B. Mycobakterien, Streptokokken, Tetanustoxoid ) und Faden-Hefe-oder Schimmel-Pilzen (z.B. Candida, Trichophyton, Mikrosporum, Aspergillus), insbesondere repräsentative Mischungen aus verschiedenen Antigenen der Gruppen (Viren, Bakterien, Pilze) oder unspezifische Stimulantien, insbesondere LPS (Lipopolysaccharide) bzw. Mitogene, vorzugsweise PWM (pokeweed mitogens) oder PHA (Phytohemagglutinin) enthält.

13. Testbesteck nach Anspruch 11 und 12, **dadurch gekennzeichnet, dass** die Musterschadstoffe aus Gemischen definierter Zusammensetzungen aus
a) aus Autoabgasen, Parfümen, Farben, Lacken, Tonern, Kohlenwasserstoffen, Lösemitteln, Insektiziden oder Holzschutzmitteln
b) aus aromatischen Kohlenwasserstoffen.
c) aus Benzol, Toluol und Xylol.
bestehen.
